# EUROPEAN PATENT SPECIFICATION

(11) **EP 1 084 402 B1**
(45) Date of publication and mention of the grant of the patent: **20.02.2002**
(21) Application number: 99926171.2
(22) Date of filing: 04.06.1999
(51) Int. Cl.: G01N 33/18, G01N 1/20, H01L 21/00

(54) **PROCESS FOR MONITORING THE CONCENTRATION OF METALLIC IMPURITIES IN A WAFER CLEANING SOLUTION**
VERFAHREN ZUR ÜBERWACHUNG DER KONZENTRATION VON METALLISCHEN VERUNREINIGUNGEN IN EINER REINIGUNGSLÖSUNG FÜR WAFER
PROCEDE DE SURVEILLANCE DE LA CONCENTRATION EN IMPURETES METALLIQUES DANS UNE SOLUTION DE NETTOYAGE DE TRANCHES

(30) Priority: 08.06.1998 US 93435
(43) Date of publication of application: 21.03.2001
(73) Proprietor: MEMC Electronic Materials, Inc., St. Peters, Missouri 63376 (US)
(72) Inventor: MORI, Erik, J., BENCCIA, CA 94510 (US); SHIVE, Larry, W., St. Peters, MO 63376 (US); SCHMIDT, Philip, R., St. Charles, MO 63303 (US)
(74) Representative: Eyles, Christopher Thomas
(86) International application number: US9912529
(87) International publication number: WO9964855

(56) References cited:
- WO-A-97/14178
- GB-A- 2 318 075
- US-A- 5 032 218
- US-A- 5 585 729
- US-A- 5 645 727
- US-A- 5 723 093

## Description

### BACKGROUND OF THE INVENTION

The process of the present invention relates generally to the cleaning of silicon wafers. More particularly, the present invention relates to a process for continuously sampling and analyzing a silicon wafer cleaning bath solution in order to determine the concentration of trace alkali, alkaline earth and transition metals contained therein.

Semiconductor wafers suitable for the fabrication of integrated circuits are produced by slicing thin wafers from a single crystal silicon ingot. After slicing, the wafers undergo a lapping process to give them a somewhat uniform thickness. The wafers are then etched to remove damage and produce a smooth surface. The next step in a conventional wafer shaping process is a polishing step to produce a highly reflective and damage-free surface on at least one face of the wafer. It is upon this polished face that electrical device fabrication takes place.

At various stages in the silicon wafer production process, such as following the polishing step, the wafer is subjected to a cleaning process in order to remove various contaminants, such as organics and other particulate contaminants, which are present on the surface of the wafer. Removal of these contaminants is typically achieved by immersing the wafer for several minutes in a series of chemical baths which act to dissolve the particulate. However, while this standard cleaning method is generally successful in removing surface particulate, it also acts to contaminate the wafer surface with metallic impurities that are present in the cleaning baths.

After polishing, silicon wafers typically have a very low surface concentration of metallic impurities. For example, wafers may have surface concentrations of metallic impurities which are as low as about 0.1 x 10¹⁰ atoms/cm². In contrast, after these wafers have undergone a standard cleaning process required to remove particulate contaminants, the surface concentrations of many of the metallic impurities may have increased to as high as about 2 to about 2.5 x 10¹⁰ atoms/cm². This re-contamination of the wafer surface is a result of the fact that, prior to the wafer being immersed in the cleaning bath solutions, the concentration of metallic impurities in such solutions is much higher than the concentration of these impurities on the wafer surface.

The presence of metallic impurities on the wafer surface can act to greatly diminish the quality and performance of integrated circuits fabricated from the wafer. As a result, integrated circuit manufacturers continues to place increasingly more stringent limitations on the surface concentrations of these impurities. For example, it is possible that within the next 5 years integrated circuit manufacturers will require that surface concentrations of metallic impurities be as low as 0.25 x 10¹⁰ atoms/cm². It is therefore important that this re-contamination of the wafer surface be prevented.

Preventing re-contamination of the wafer surface means wafer cleaning solutions must be prepared to have far lower concentrations of such metals than exist presently. In order to prepare such cleaning solutions, the sources of these impurities must be identified and eliminated. This means that each reagent used in the preparation of a particular cleaning solution must be analyzed and the concentration of these metals determined. However, successfully identifying the source of a metallic impurity is dependent upon the accuracy and reliability of the analytical data that is obtained. In addition, because even very low levels of a metal can results in wafer surface contamination, the sensitivity of the analytical method is also critical.

Traditionally, the type and concentration of a metallic impurity in a cleaning bath solution has been monitored through the use of a manual sampling technique, commonly referred to as an "off-line" method of sampling, wherein a human operator collects a sample of the cleaning bath solution. The sample is then transported to a laboratory for analysis. There are, however, several disadvantages to this "off-line" method of sampling and analysis of the cleaning bath solutions.

One disadvantage of this off-line method is that it is prone to the introduction of additional contaminants from outside sources. For example, human contact with the sample often leads to the introduction of metallic contaminants such as aluminum, iron, calcium, and sodium. In addition, the vial or container which holds the collected sample, as well as the pipette or other sampling device used to collect the sample, typically cannot be sufficiently cleaned such that the introduction of outside contaminants into the sample is avoided. As a result, experience has shown that it is generally not possible to accurately and reproducibly detect metal concentrations at levels which are less than about 100 parts per trillion (ppt).

In addition to the difficulties in obtaining accurate and reproducible results, due to the likelihood of outside contamination, this off-line method also lack the sensitivity needed in order to accurately detect metal concentrations of less than about 100 ppt. Since this conventional method lacks the ability to detect the presence of metals at such low concentrations, the wafer may still be contaminated by metallic impurities even though, based on the results obtained from such a conventional analysis, the solution appears to be acceptable. Such poor sensitivity also makes it difficult to determine the source of such impurities, as well.

Another disadvantage of this off-line method is that it can take several hours for a laboratory to complete the analysis of the sample and provide the results to those in the wafer production area who are responsible for wafer cleaning. In the interim period, if wafer cleaning continues, a bath that is ultimately found to be contaminated can produce hundreds of unacceptable wafers; when this occurs, the wafers must be recalled and cleaned again before further processing can occur. To avoid such problems, use of the bath may be halted while the operator waits for the results. In either case, the net effect is an increase in production cost and a decrease in overall efficiency of the cleaning process, due to the introduction of such unnecessary delays.

To avoid the time consuming and potentially costly process of off-line laboratory sampling and analysis, it has become common practice to simply discard the cleaning solution after a pre-determined period of time, such as about every 2 to 6 hours. However, the many variables which dictate the useful life of a cleaning bath solution, including the quality of the chemical reagents, the quality of the process water, and the precautions taken to prevent contamination by human operators, are not taken into account by such an approach. Therefore, without the benefit of an analysis for metallic impurities, a portion of the useful life of the bath is likely wasted. Discarding solutions before it becomes necessary also increases production costs due to the lost chemicals and the increase in the amount of waste solution that must be handled and removed.

In view of the forgoing, a need continues to exist for a process for determining the concentration of metallic impurities in a wafer cleaning solution which does not act to delay wafer cleaning, which does not act to contaminate the sample, and which allows for the reliable, reproducible, and accurate detection of such impurities at level far below 100 ppt.

### SUMMARY OF THE INVENTION

Among the objects of the present invention, therefore, are the provision of a process which allows for continuous sampling of the cleaning bath solution; the provision of such a process which efficiently determines the concentration of metallic contaminants in the cleaning solution in near real time; the provision of such a process which enables the efficient detection of wafers having unacceptable surface concentrations of metallic contaminants by means of analyzing the cleaning solutions in which the wafers have been treated; and, the provision of such a process which maximizes the useful life of a cleaning bath solution by efficiently determining the concentration of metallic contaminants contained therein.

Briefly, therefore, the present invention is directed to a process for determining the concentration of a metallic impurity or impurities in a silicon wafer cleaning solution, the solution being maintained in continuous flow through a bath. The process comprises continuously delivering from said flow a portion of the cleaning bath solution to and through a sample cell. A sample of the solution is then withdrawn a from the sample cell and analyzed in order to determine the concentration of trace alkali, alkaline earth or transition metal impurity or impurities in the cleaning bath solution. The cleaning solution delivered through the sample cell is discarded.

The present invention is further directed to a process for determining whether the concentration of a metallic impurity or impurities on a surface of a silicon wafer exceeds an acceptable limit. The process comprises immersing the silicon wafer in a cleaning solution which is maintained in continuous flow through a bath. A portion of this flow of solution is continuously delivered to and through a sample cell. A sample of the solution is then withdrawn from the sample cell and analyzed to determine the concentration of trace alkali, alkaline earth or transition metal impurity or impurities in the cleaning bath solution. A comparison is then made between the concentration of impurities in the cleaning solution and an empirically determined correlation in order to determine if the concentration of impurity or impurities on the silicon wafer surface exceeds about 1 x 10¹⁰ atoms/cm².

Other objects and features of this invention will be in part apparent and in part pointed out hereinafter.

### BRIEF DESCRIPTION OF THE DRAWINGS

FIG. 1 is a schematic view of an on-line wafer cleaning system, including on-line sample collector and analytical instrumentation, in accordance with the process of the present invention.

FIG. 2 is a perspective view of a preferred embodiment of a sample collector, or cell.

FIG. 3 is a sectional view taken along the plane of line 3-3 of FIG. 2.

FIG. 4 is a sectional view taken along the plane of line 4-4 of FIG. 2.

FIG. 5 is a plot showing a correlation between the log of the concentration of sodium in a SC-2 cleaning bath solution and the log of the concentration of sodium, minus 10, on the surface of a number of silicon wafers.

FIG. 6 is a plot showing a correlation between the log of the concentration of nickel in a SC-2 cleaning bath solution and the log of the concentration of nickel, minus 10, on the surface of a number of silicon wafers.

Corresponding reference characters indicate corresponding parts throughout the drawings.

### DETAILED DESCRIPTION OF THE PREFERRED EMBODIMENTS

In accordance with the present invention, the concentration of metallic impurities in a cleaning bath solution is determined by an on-line process which allows for the sampling and analysis of the cleaning solution in a substantially metal-free environment. It is to be noted that, as used herein, the term "on-line" shall refer to a process wherein solution sampling and analysis occurs as a single, integrated process in conjunction with wafer cleaning. Stated another way, the term "on-line" shall refer to a process wherein a sample may be removed from a wafer cleaning solution and analyzed without being exposed to direct, or intimate, contact with a human operator or other outside source of metallic impurities. It is also to be noted that, as used herein, the phrase "substantially metal-free environment," as well as variations thereof, shall refer to a process wherein solution sampling and analysis may be performed such that an accurate and reproducible detection limit for alkali, alkaline earth and transition metals of less than about 100 parts per trillion (ppt), and as low as about 1 ppt, may be achieved. Stated another way, this phrase shall refer to a process wherein solution sampling and analysis may be performed in such a way that the sample is not exposed to outside sources of metallic impurities, such as sample collection containers or human operators, thus allowing for concentrations of metallic impurities as low as about 1 ppt to be accurately and reproducibly detected. It is to be further noted that the term "automated," as well as variations thereof, shall refer to a process which is capable of being substantially performed without the aid of human operators. Stated another way, the term "automated" as used herein shall refer to a process for sampling and analyzing a cleaning bath solution which generally requires the involvement of a human operator only to routinely calibrate the instrumentation.

Conventionally, organic and other particulate contaminants are removed from the surfaces of a single crystal silicon wafer by immersing the wafer for several minutes in each of a series of cleaning bath solutions. Unfortunately, while this method is successful in removing such contaminants, it also results in the deposition of metallic impurities which are contained within the cleaning solutions onto the surface of the wafer. The process of the present invention provides a way to more accurately and consistently identify the source or sources of these impurities so that they can be eliminated, thus affording the means by which to prepare cleaning solutions which are substantially free of such impurities. For example, the present process may be used to test reagents directly, prior to their use in the cleaning solutions, thus aiding in the preparation of solutions which are substantially metal-free (i.e., solutions having metal concentrations which are less than about 100 ppt, preferably less than about 50 ppt, more preferably less than about 25 ppt, still more preferably less than about 10 ppt, and most preferably less than about 1 ppt). In addition, once such cleaning solutions have been prepared, the process of the present invention affords the means by which to continually monitor each of the cleaning solutions in use to more consistently and efficiently determine the concentration of metallic impurities, such as alkali, alkaline earth or transition metals. In this way the use of a particular cleaning solution, once it becomes unacceptably contaminated, can be halted before wafers are contaminated by the solution and further processed.

The process of the present invention involves the use of on-line instrumentation for sampling and analysis. This process affords more accurate and reliable results because it significantly reduces the potential for contamination of the sample to be analyzed from outside sources, such as through direct human contact or through the use of sampling devices or containers which are contaminated. This process also allows for a significant reduction on the time required to sample and analyze a cleaning solution, as compared to existing "off-line" methods wherein the sample is collected and transported to another location, often a site not proximate to the wafer production area, in order for the analysis to be performed.

In a typical wafer cleaning process, a wafer is immersed in a series of cleaning bath solutions. These cleaning solutions include SC-1 (a solution comprising ammonium hydroxide, hydrogen peroxide and water, typically in a 1:1:5 ratio), SC-2 (a solution comprising water, hydrogen peroxide and hydrochloric acid, or citric acid, in a 5:1:1 ratio), as well as other acidic solutions (including solution of hydrogen fluoride, nitric acid, sulfuric acid, and phospnoric acid), ozonated water, as well as deionized water for rinsing. Each cleaning bath solution in a series is typically recirculated continuously, by means of a recirculation line, filter and pump, while a wafer, or cassette of wafers, is immersed in the solution. Recirculation through a filter aids in the removal of organic and other particulate contaminants from the solution and helps prevent such contaminants from being redeposited on the wafer surface.

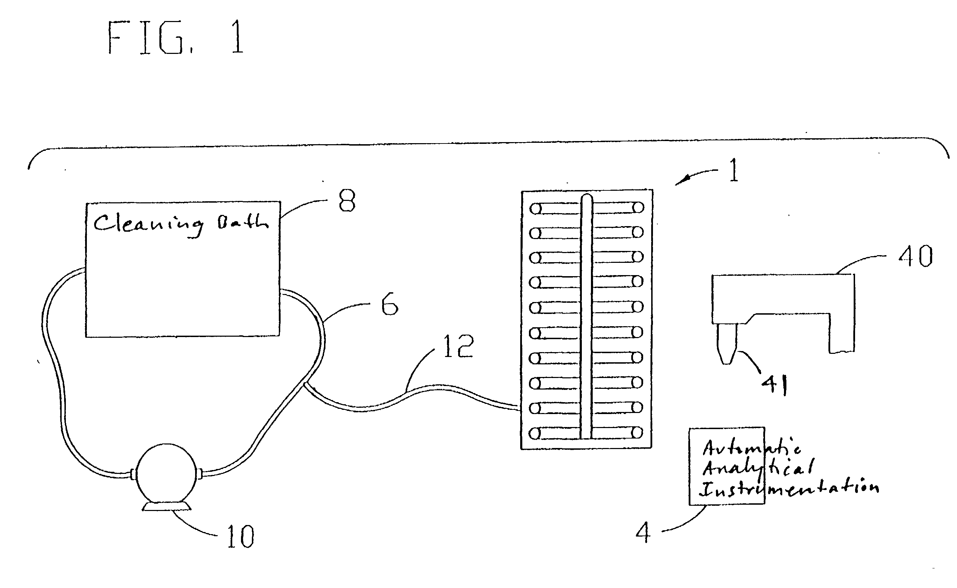

Referring now to Fig. 1, in a preferred embodiment of the present process, on-line sampling and analysis are achieved by connecting in series, by means common to the art, a sampling cell or collector, indicated generally at **1**, an analytical instrumentation **4**, and a cleaning bath **8**; the sampling cell, analytical instrument and cleaning bath each being proximate to the others. The collector **1** is connected to a recirculation line **6** of the cleaning bath **8** by a suitable bleed line **12**. As recirculation pump **10** continuously circulates the cleaning solution through the cleaning bath, a small portion of the cleaning solution in the cleaning bath is bled off into the bleed line **12** for delivery to the collector **1**.

It is to be noted in this regard that while such a configuration is preferred, suitable alternatives are possible and are intended to be within the scope of the present invention. For example, the sample may be obtained either directly from the cleaning bath (i.e., the bleed line **12** may be directly plumbed from the bath **8** to the analytical instrument **4**, with suitable valving in order to control flow as necessary), or from any position along the recirculation line **6** (i.e., the bleed line **12** may be connected at any position to the recirculation line **6**).

The bath which contains the cleaning solution, the recirculation and bleed lines, as well as all the parts of the pump **10**, collector **1** and analytical instrument **4** which contact the solution are preferably constructed, lined or coated with a material which, after thorough cleaning, will not leach metallic impurities into the solution and, therefore, cause inaccurate and inconsistent results to be obtained upon analysis of the solution. For example, the bath is typically constructed of quartz, while the bleed line **12** is preferably constructed from Teflon (i.e., polytetrafluoro-ethylene). Alternatively, however, the bleed line may be constructed from poly(vinylidene fluoride) or other comparable materials, depending upon the composition of the cleaning solution being sampled and analyzed. For example, high purity polypropylene has been found to be suitable for use with acidic solutions in which hydrochloric acid is the acidic component.

The cleaning solution may be diverted through the bleed line **12** either continuously or intermittently. If the solution is to be diverted intermittently, a valve (not shown) may be inserted between the recirculation line 6 and the bleed line **12**. Preferably, however, the solution is diverted continuously in order to ensure that at all times the solution being fed to the collector **1** is representative of the solution within the bath **8**. This continuous flow of solution enables near real time analysis of the cleaning solution of interest; that is, the continuous flow ensures that the sample will, at the moment of collection and analysis, be representative of the solution in the cleaning bath itself, Furthermore, such a continuous flow acts to minimize or prevent contamination of the solution itself as a result of leaching of metallic impurities from the bleed line **12** or collector **1**, which may occur if the solution is allowed to become stagnant.

To avoid the possibility of contaminants becoming re-introduced into the cleaning solution, typically the solution that is diverted through the bleed line **12** is not returned to the cleaning bath. As a result, in order to avoid the excessive depletion of cleaning solution from the bath, the diameter of the bleed line is preferably as small as the system configuration and process conditions will allow. Typically, the diameter of the bleed line is less than about 6.350 mm (1/4 in.)in diameter, preferably about 3.175 mm(1/8 in.)in diameter, and more preferably about 1.588 mm (1/16 in.) in diameter.

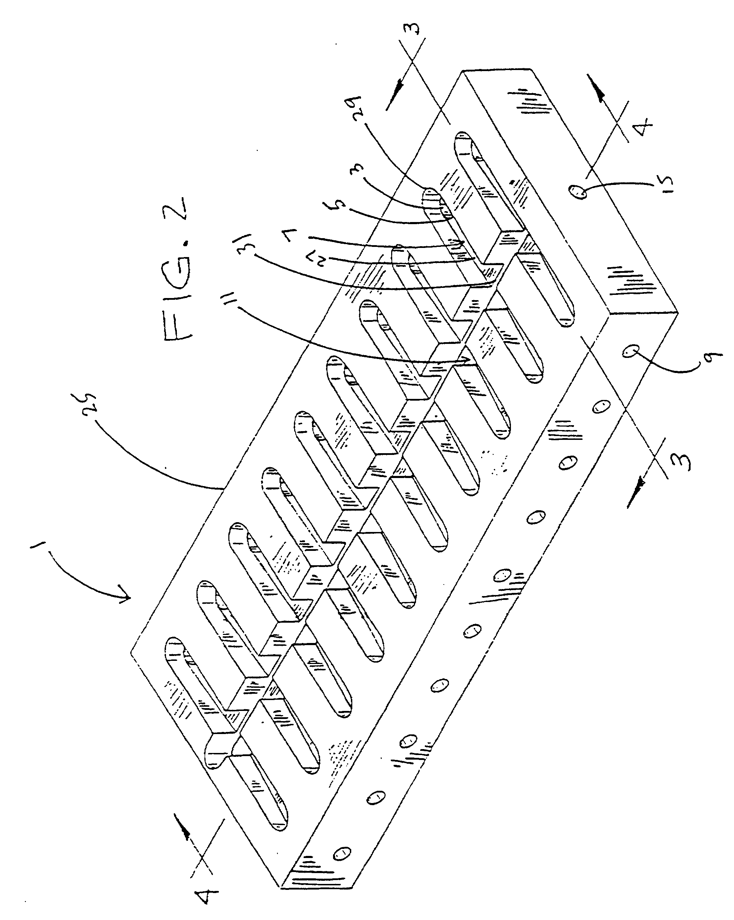

Referring now to Figs. 2-4, a preferred embodiment of the collector **1** will be further described. Note that a more detailed description of this preferred embodiment may be found in International Patent Publication No. WO99/64838. The collector **1** comprises a generally rectangular block **25** constructed from, as noted above, a material which will not leach metallic impurities into the solution after having been thoroughly cleaned, For example, the block **25** of the illustrated embodiment is preferably constructed from Teflon. It is to be noted, however, that materials having similar properties may also be used without departing from the scope of this invention. It is to be further noted that the form, shape, or design of the collector may differ from the preferred embodiment disclosed herein without departing from the scope of this invention.

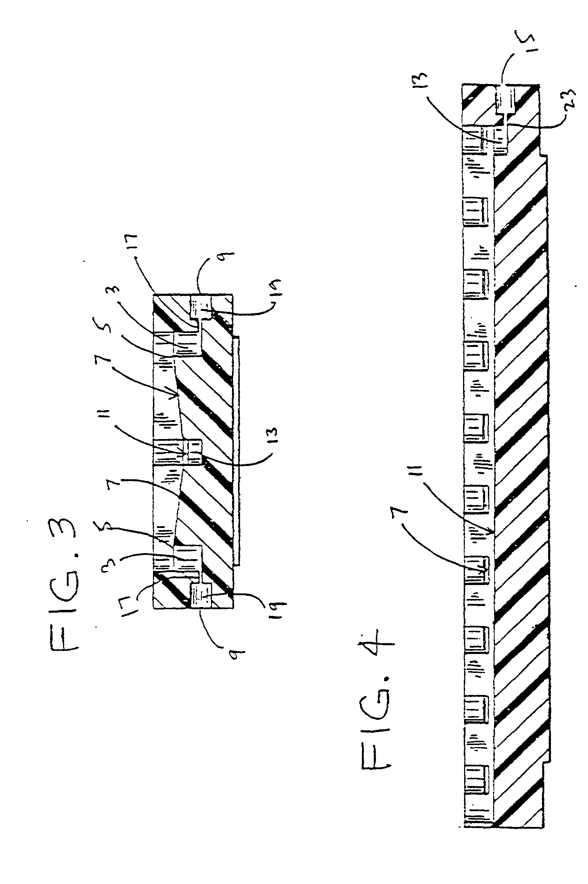

The block **25** has a central waste trough **11** extending longitudinally within the upper surface of the block and multiple receptacle overflow spillways or troughs **7** extending laterally within the upper surface of the block in communication with the central waste trough. The waste trough **11** and spillways **7** are designated generally by their reference numbers. The receptacle spillways **7** each have a floor **27**, an outer end **29** disposed generally adjacent a lateral edge margin of the upper surface of the block and an inner end **31** opening into the central waste trough **11**. The floor **27** of each receptacle spillway **7** slopes downward from the outer end **29** to the inner end **31** of the receptacle trough for delivering liquid in the receptacle trough to the central waste trough **11** (Fig. 3).

Vertically oriented receptacles **3** are formed in the block at the laterally outer end **29** of each spillway **7**. The receptacles **3** open at their upper ends into the spillways **7** to allow overflow liquid from the receptacles to drain to the central waste trough **11**. The diameter of each receptacle is sized to permit a sample collector **41** of the automated analytical instrumentation **4** (Fig. 1) to be dipped down into the receptacle **3** for collecting a sample to be analyzed. Each receptacle **3** also has an inlet **17** generally adjacent the bottom of the receptacle for receiving fluid into the receptacle (Fig. 3). The inlet **17** communicates with a respective inlet port **9** in a side wall of the block. The diameter of the inlet **17** is substantially reduced at an inner portion adjacent the receptacle for controlling the flow rate of liquid into the receptacle. The inlet port **9** communicates with the cleaning bath **8** (Fig. 1) by the bleed line **12** for receiving the liquid into the collector **1** to analyze the liquid.

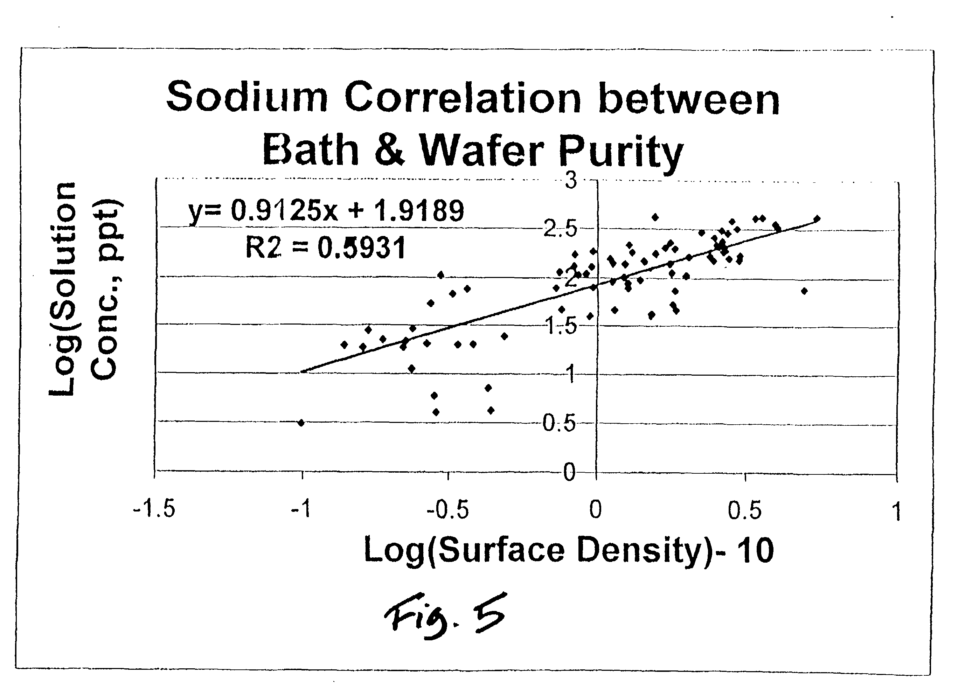

With particular reference to Fig. 4, the central waste trough **11** slopes downward from a rear end of the block **25** toward a front end of the block to direct liquid in the waste trough **11** to a waste drain **13**. The waste drain **13** has an outlet **23** generally adjacent the bottom of the waste drain for discharging liquid from the waste drain. The outlet **23** extends to an outlet port **15** in the front end of the block **25** for discharging liquid from the collector **1** to a suitable drainage system (not shown) or sewer (not shown) of the type well known to those of ordinary skill in the art.

Analysis of the sample, once collected, is achieved using instrumentation common in the art for detecting trace amounts of metallic impurities. Such instrumentation includes atomic absorption, inductively-coupled plasma mass spectrometry (ICP/MS), capillary electrophoresis, and ion chromatography, among others. Common metallic impurities of particular interest include alkali, alkaline earth and transition metals, such as sodium, nickel, iron, copper, aluminum, zinc, and calcium, as well as titanium, potassium, cobalt, chromium, molybdenum and manganese. The precise instrument selected is in part a function of the sensitivity of the instrument for the particular metallic impurity, or impurities, for which a cleaning solution is being monitored. For example, often a rinse bath, containing only deionized water, will be at the end of a cleaning sequence. Such a bath may be connected to a capillary electrophoresis or an ion chromatography instrument in order to monitor the concentration of, among other things, chloride, fluoride or ammonium ions, as well as sulfates or nitrates.

In a preferred embodiment, the analytical instrument **4** (Fig. 1) is automated, possessing a sample collecting device **40** and a syringe, or other suitable sampling device, **41** which may be inserted into the receptacle **3** through its opening **5** by a robotic arm or similar automated mechanism. Again through automation, the syringe extracts a sample of the cleaning solution from the receptacle. The syringe is then withdrawn from the receptacle and the contents of the syringe are analyzed. Common automated instrumentation which are acceptable for use as the collecting device **40** include an autosampler designed for ICP/MS instrumentation (e.g., Cetac 500, commercially available from Cetac of Omaha; Gilson 222, commercially available from Gilson of England). Common analytical instrumentation **4** include a HP4500 ICP/MS (commercially available from Hewlitt Packard; Sunnyvale, CA), and a Varian 400 AA (commercially available from Varian; Sunnyvale, CA).

An automated analytical instrumentation is preferred because it allows for the more frequent analysis of a given cleaning bath solution. For example, using a conventional sampling and analysis method in a typical wafer production facility, it is not uncommon for a cleaning solution to be sampled and analyzed only about once over the course of a standard eight hour production shift, if at all. In contrast, the process of the present invention allows for such a solution to be sampled and analyzed on a much more frequent basis. For example, using a preferred embodiment of the collector **1** generally allows for essentially continuous sampling of a cleaning bath solution. Likewise, through means of automation, analysis of the solution may be performed as frequently as the analytical instrumentation of choice, and the design or configuration of the system, will allow. However, typically a solution will be sampled and analyzed using the present process on an interval of about 60 minutes to about 90 minutes, preferably about 30 minutes to about 50 minutes, more preferably about 15 minutes to about 25 minutes, and most preferably about 5 minutes to about 10 minutes. It is to be noted, however, that the frequency with which a solution is tested may be other than that herein described without departing from the scope of the present invention.

In a preferred embodiment, the collector **1** comprises more than one receptacle so that a plurality of cleaning baths and/or cleaning lines can be connected to the collector at the same time. As shown in Fig. 2, the collector **1** has two sets of ten receptacles **3**, one set being on each side of the central waste trough **11.** The receptacles **3** of each set are arranged in a series, which in the illustrated embodiment is a line of equally spaced receptacles, to permit the instrumentation **4** to progressively dip into the receptacles one after another in a predetermined order. Each receptacle **3** would be connected to its own bleed line (not shown) in order to receive liquid from a different cleaning bath (not shown). It is understood, however, that the arrangement and number of receptacles **3** in the collector **1** may vary without departing from the scope of this invention.

In operation, the recirculation pump **10** draws cleaning bath solution from the cleaning bath **8** and pumps the solution through the recirculation line **6**. The recirculation pump **10** creates sufficient pressure in the recirculation line **6** such that a small portion of the cleaning solution is continuously diverted from the recirculation line, through the bleed line **12** for delivery to the collector **1**. The solution then flows through the inlet port **9** of the block **25** into the inlet **17**. The inlet **17** meters the solution into the receptacle **3**. Since the solution diverted through the inlet **17** is typically not returned to the cleaning bath **8**, the diameter of the inlet is preferable minimized to prevent excessive depletion of the cleaning solution from the bath. For example, the inlet **17** of the illustrated embodiment is preferably less than about one-quarter inch (about 6.350 mm) in diameter, and more preferably less than about oneeighth inch (about 3.175 mm) in diameter.

The receptacle inlet **17** delivers liquid to the receptacle **3** at the bottom of the receptacle so that the liquid flows upward from the bottom of the receptacle. Filling the receptacle **3** from the bottom prevents stagnation of the solution in the receptacle **3**. Further, by eliminating stagnation of the cleaning solution, the solution within the receptacle **3** at all times remains representative of the cleaning solution contained within the cleaning bath **8**. Moreover, any impurities which might be introduced from the syringe **41** of the sample collector device **40** are prevented from entering the cleaning bath **8**.

A continuing flow of solution into the receptacle **3** causes the solution to overflow the receptacle into the spillway **7**. The sloped floor **27** of the spillway **7** cirects the overflow solution downward, away from the receptacle **3** and into the waste trough **11.** The waste trough **11** receives the overflow liquid and directs the waste solution to the waste drain **13**. The overflow solution exits the waste drain through the outlet **23**, and is ultimately discharged from the block **25** to a suitable drainage system or sewer via the outlet port **15.**

The syringe **41**, or other sampling means of the automated analytical instrumentation **4**, is selectively inserted into the upper end of one of the receptacles 3 by a robotic arm **40** or the like. The syringe automatically extracts a sample of the cleaning solution from the receptacle and then is withdrawn. The contents of the syringe **41** are then injected into the automated analytical instrumentation **4** for analysis.

By utilizing on-line sampling and analysis, the process of the present invention acts to eliminate potential sources of outside contamination. Experience to-date has shown that the present process therefore allows for more consistent and reproducible results to be obtained. For example, if a conventional method of sampling and analysis is used, experience has shown that results which indicate the concentration of a given metallic impurity is less than 100 ppt are suspect. Generally speaking, such results from a conventional method would likely prompt one to obtain another sample and perform the analysis again for confirmation.

In contrast, the process of the present invention enables one to consistently and reproducible detect metallic impurities at concentration of less than about 100 ppt, preferably less than about 50 ppt, more preferably less than about 25 ppt, still more preferably less than about 10 ppt, and most preferably less than about 1 ppt. The reproducibility and consistency of the results obtained from the present process are due to a number of features. First, the equipment is constructed of high purity materials. Second, automation allows for the elimination of most common outside sources of contamination. Third, the very nature of such a "closed system" allows for in-situ cleaning, which generally enables a more thorough and consistent cleaning of the system before wafer cleaning begins. For example, if the system becomes contaminated with sodium, the system may be drained and repeatedly flushed with deionized water until the water tests below the desired upper limit. Likewise, if the system becomes contaminated with iron, a strongly acid solution, such as a solution of nitric acid, may be used to clean and flush the system.

It is to be noted in this regard that the solution which is used to flush the system is in part a function of the solubility of the metal, or metals, to be removed. In addition, materials of construction will also factor into determining what type of cleaning solution, as well as the cleaning sequence, is needed. For example, if particular components of the system are fabricated of Teflon, an extremely pure, concentrated acid will need to be heated and recirculated through the system to leach out and remove any metals which are present. This is primarily due to the porous nature of the material. In contrast, quartz is not porous and, therefore, may be much more easily cleaned.

Finally, more consistent and reproducible results may be obtained as a result of the continuous flow of the solution to be analyzed into and through the sample collector or cell. As noted, such a process acts to prevent stagnation and, thus, prevent the possibility of contamination from any leaching of metallic impurities which may otherwise occur. Furthermore, this continuous flow enables an "equilibrium" of sorts to be reached between the system itself and the solution which is to be analyzed. This is an important feature because often the system will initially be cleaned with a solution having a composition different from the composition of the wafer cleaning bath solution to be analyzed. As a result, it is possible that certain metallic impurities may be left behind after the initial system cleaning is complete. Such impurities may then become solubilized or suspended in the wafer cleaning solution and cause inaccurate results to be obtained.

In addition to affording a means by which to monitor a cleaning bath solution on a more frequent and efficient basis, and to consistently detect metallic impurities at concentrations not here-to-for attainable by conventional methods of sample collection and analysis, the present invention also affords the means by which to determine whether the concentration of a particular metallic impurity on the surface of a silicon wafer exceeds some predetermined limit. As a result, the acceptability of the wafer may be evaluated. Thus, the present invention affords an alternative to standard vapor phase decomposition (VPD) or acid drop methods of analysis. (See, e.g., U.S. Pat. Nos. 5,633,172 and 4,990,459.)

Generally, VPD and acid drop methods of analysis involve the application of an acid onto the surface of the silicon wafer in order to dissolve metallic impurities which are present. The surface of the wafer is rinsed, and then the rinse water is collected and analyzed by conventional means, in order to identify and determine the concentration of metallic impurities contained therein. The resulting metallic impurities present are attributable to the wafer surface.

As an alternative to these conventional methods, in accordance with another preferred embodiment of the present invention, the concentration of metallic impurities on the surface of a wafer may be approximated by analyzing the appropriate wafer cleaning solution in which the wafer has been treated. Stated another way, by analyzing a given cleaning solution, a correlation may be made between the concentration of the metallic impurity in the cleaning solution and the concentration of the metallic impurity on the wafer surface. Generally, this correlation may be empirically determined by first treating a number of silicon wafers in a given cleaning solution. The solution may then be tested using the on-line process of the present invention, while each wafer may is analyzed using the standard acid drop method. A correlation may then be made between the concentration of the metallic impurity in the cleaning solution and the concentration of the same impurity on the wafer surface. As the concentration of the metallic impurity increases in the solution, eventually a level will be reach which results in the production of a wafer which is contaminated with an unacceptably high surface concentration of the specific metallic impurity of interest. By correlation of the solution concentration with the surface concentration of the wafer, an observation may thus be made that when the cleaning solution concentration exceed this particular limit, all wafers subsequently cleaned in the solution will be unacceptably contaminated. Stated another way, the observation may be made that as long as the concentration of the particular metallic impurity of interest in the solution is below the targeted limit, each wafer that is cleaned in the solution will contain an acceptable concentration of that metallic impurity.

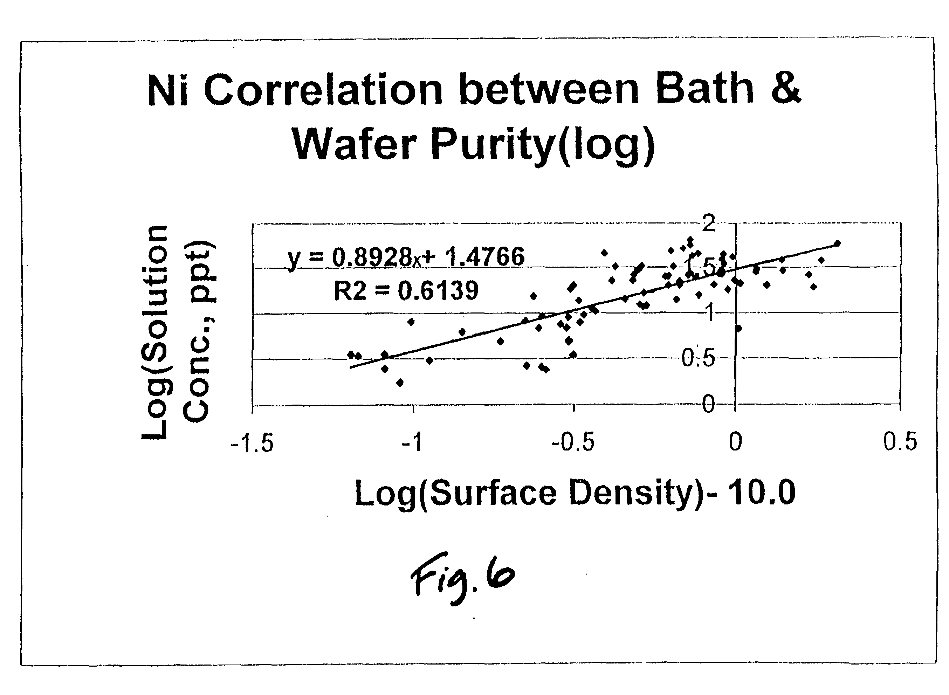

In this regard, referring now to Figs. 5 and 6, such an empirically determined correlation may be shown for sodium and nickel, respectively, in a SC-2 bath which is part of a standard cleaning sequence. In this process, the bath was configured for continuous recirculation or flow. Multiple samples of the solution were collected and analyzed using the present process. Likewise, multiple samples were taken from the wafers which were processed through this bath and analyzed using a conventional acid drop method. The data collected from these analyses was then used to plot the log of the concentration (in ppt) of sodium and nickel in the rinse bath versus the log of the concentration (in atoms/cm²), minus 10, of these metals on the surfaces of the wafers (see Figs. 5 and 6).

In reviewing these plots, it can be seen that a "best-fit" line may be drawn and used to establish a correlation between the concentration of sodium or nickel in the rinse bath and the concentration of sodium or nickel on the surfaces of the wafers. As a result, observations may be made about the surface concentration of these metals given a particular solution concentration. For example, following the best-fit line of Fig. 5, it may be observed that when the concentration of sodium in the solution is about 100 ppt, the concentration of sodium on the surface of a wafer is about 1.2 x 10¹⁰ atoms/cm², while a solution concentration of about 25 ppt correlates to a surface concentration of about 0.3 x 10¹⁰ atoms/cm². Likewise, following the best fit line of Fig. 6, it may be observed that when the concentration of nickel in the solution is about 25 ppt, the concentration of nickel on the surface of a wafer is about 1 x 10¹⁰ atoms/cm², while a solution concentration of about 10 ppt correlates to a surface concentration of about 0.3 x 10¹⁰ atoms/cm².

Accordingly, by means of this correlation data, the acceptability or unacceptability of a wafer may be determined by sampling and analyzing a given cleaning solution. It is to be noted in this regard that the "acceptability" or "acceptability" of a wafer is dependent upon the specific end application, as well as the limitations imposed by integrated circuit manufactures for a given metallic impurity. For example, typical integrated circuit manufacturers have imposed limitations which currently require the surface concentration of metals such as aluminum and calcium not exceed about 1 x 10¹¹ atoms/cm². The limitation on most other metals is currently about 1 x 10¹⁰ atoms/cm². Preferably, however, it is desirable that the concentration of metallic impurities not exceed about 0.75 x 10¹⁰ atoms/cm², more preferably about 0.5 x 10¹⁰ atoms/cm², and still more preferably about 0.25 x 10¹⁰ atoms/cm². Most preferably, it is desirable that the concentration of such impurities not exceed about 0.1 x 10¹⁰ atoms/cm².

However, it is also to be noted that this same empirical process may be employed to determine the maximum concentration of a metallic impurity within a given bath that is acceptable in order to ensure that the concentration of this same impurity on the wafer surface does not exceed the desired limit.

In addition to providing information as to the concentration of metallic impurities on the wafer surface, the present process may also afford the means by which to extend the useful life of a given cleaning solution. The useful life a cleaning solution may be extended, once such a correlation is determined, because now the solution may remain in the cleaning bath sequence until, as noted above, it becomes unacceptably contaminated. For example, rather than simply discarding the solution after about 2 to 6 hours, use of the solution may continue for about 8 hours, 10 hours, 12 hours, 14 hours or more.

As a result of extending the useful life of the cleaning solution, production costs may be reduced, as well as the amount of waste solution generated, because the solution will no longer simply be discarded after an arbitrarily determined period of time. Accordingly, depending upon the concentration of impurities which are acceptable on the wafer surface, a cleaning solution may be monitored using the process of the present invention and allowed to remain in use until the level or concentration of metallic impurities in the solution is found to be in excess of about 100 ppt, preferably about 75 ppt, more preferably about 50 ppt, and still more preferably about 25 ppt. In order to ensure the preparation of wafers having very low surface concentrations of metallic impurities, most preferably the solution is monitored and allowed to remain in use only until the concentration of metallic impurities in solution exceeds about 10 ppt.

In view of the above, it will be seen that the several objects of the invention are achieved.

As various changes could be made in the above-described process without departing from the scope of the invention, it is intended that all matter contained in the above description be interpreted as illustrative and not in a limiting sense.

## Claims

1. A process for determining the concentration of a metallic impurity or impurities in a silicon wafer cleaning solution which is maintained in continuous flow through a bath, the process comprising:
continuously delivering from said flow a portion of the cleaning bath solution to and through a sample cell;
withdrawing a sample of the cleaning solution from the sample cell;
analyzing the withdrawn sample to determine the concentration of a trace alkali, alkaline earth or transition metal impurity or impurities in the cleaning bath solution; and
discarding the cleaning solution delivered through the sample cell.

2. A process according to claim 1, wherein the trace alkali, alkaline earth or transition metal impurity or impurities for which the withdrawn sample is analyzed is or are selected from sodium, nickel, iron, copper, aluminum, zinc, calcium, titanium, potassium, cobalt, chromium, molybdenum and manganese.

3. A process according to claim 1 or claim 2, wherein analysis of the withdrawn sample is automated.

4. A process according to any one of claims 1 to 3, wherein the sample cell is proximate to the analytical instrumentation, such that the sample may be withdrawn and analyzed without transporting the sample cell to the analytical instrumentation.

5. A process according to any one of claims 1 to 4, wherein a sample is withdrawn from the sample cell and analyzed at intervals of about 5 to about 10 minutes.

6. A process for determining whether the concentration of a metallic impurity or impurities on a surface of a silicon wafer exceeds an acceptable limit, the process comprising:
immersing a silicon wafer in a cleaning solution which is maintained in continuous flow through a bath;
continuously delivering from said flow a portion of the cleaning bath solution to and through a sample cell;
withdrawing a sample of the cleaning solution from the sample cell;
analyzing the withdrawn sample to determine the concentration of trace alkali, alkaline earth or transition metal impurity or impurities in the cleaning bath solution; and,
comparing the concentration of impurity or impurities in the cleaning solution to an empirically determined correlation in order to determine if the concentration of impurity or impurities on the silicon wafer surface exceeds about 1 x 10¹⁰ atoms/cm².

7. A process according to claim 6, wherein sample collection and analysis occurs while the wafer is immersed in the solution.

8. A process according to claim 6 or claim 7, wherein the comparison between the impurity or impurities in the cleaning solution to the empirically determined correlation is made in order to determine if che concentration of impurity or impurities on the silicon wafer surface exceeds about 0.5 x 10¹⁰ atoms/cm².

9. A process according to any one of claims 6 to 8, wherein the trace alkali, alkaline earth or transition metal impurity or impurities for which the withdrawn sample is analyzed is or are selected from sodium, nickel, iron, copper, aluminum, zinc, calcium, titanium, potassium, cobalt, chromium, molybdenum and manganese.

10. A process according to any one of claims 6 to 9, wherein analysis of the withdrawn sample is automated.

## Patentansprüche

1. Verfahren zum Bestimmen der Konzentration einer metallischen Verunreinigung oder metallischer Verunreinigungen in einer Siliziumscheiben-Reinigungslösung, die in kontinuierlichem Strom durch ein Bad gehalten wird, bei dem man
einen Teil der Reinigungsbadlösung von dem genannten Strom kontinuierlich zu einer und durch eine Probenzelle leitet,
eine Probe der Reinigungslösung aus der Probenzelle abzieht,
die abgezogene Probe analysiert, um die Konzentration einer Spurenverunreinigung oder von Spurenverunreinigungen an Alkali-, Erdalkali oder Übergangsmetall in der Reinigungsbadlösung zu bestimmen, und
die durch die Probenzelle geleitete Reinigungslösung verwirft.

2. Verfahren nach Anspruch 1, bei dem die Spurenverunreinigung oder -verunreinigungen an Alkali-, Erdalkali- oder Übergangsmetall, auf die die abgezogene Probe analysiert wird, unter Natrium, Nickel, Eisen, Kupfer, Aluminium, Zink, Calcium, Titan, Kalium, Kobalt, Chrom, Molybdän und Mangan ausgewählt wird oder werden.

3. Verfahren nach Anspruch 1 oder Anspruch 2, bei dem die Analyse der abgezogenen Probe automatisiert ist.

4. Verfahren nach einem der Ansprüche 1 bis 3, bei dem die Probenzelle der analytischen Instrumentierung benachbart ist, so dass die Probe abgezogen und analysiert werden kann, ohne dass die Probenzelle zu der analytischen Instrumentierung transportiert wird.

5. Verfahren nach einem der Ansprüche 1 bis 4, bei dem eine Probe in Intervallen von etwa 5 bis etwa 10 Minuten aus der Probenzelle abgezogen und analysiert wird.

6. Verfahren zur Bestimmung, ob die Konzentration einer metallischen Verunreinigung oder metallischer Verunreinigungen auf einer Oberfläche einer Siliziumscheibe eine zulässige Grenze überschreitet, bei dem man
eine Siliziumscheibe in eine Reinigungslösung eintaucht, die in kontinuierlichem Strom durch ein Bad gehalten wird,
einen Teil der Reinigungsbadlösung aus dem Strom kontinuierlich zu einer und durch eine Probenzelle leitet,
eine Probe der Reinigungslösung aus der Probenzelle abzieht,
die abgezogene Probe analysiert, um die Konzentration von Spurenverunreinigung oder -verunreinigungen an Alkali-, Erdalkali- oder Übergangsmetall in der Reinigungsbadlösung zu bestimmen, und
die Konzentration der Verunreinigung oder Verunreinigungen in der Reinigungslösung mit einer empirisch bestimmten Korrelation vergleicht, um zu bestimmen, ob die Konzentration der Verunreinigung oder Verunreinigungen auf der Oberfläche der Siliziumscheibe etwa 1 x 10¹⁰ Atome/cm² übersteigt.

7. Verfahren nach Anspruch 6, bei dem die Probensammlung und die Analyse erfolgen, während die Scheibe in die Lösung eingetaucht ist.

8. Verfahren nach Anspruch 6 oder Anspruch 7, bei dem der Vergleich zwischen der Verunreinigung oder den Verunreinigungen in der Reinigungslösung mit der empirisch bestimmten Korrelation erfolgt, um zu bestimmen, ob die Konzentration der Verunreinigung oder Verunreinigungen auf der Oberfläche der Siliziumscheibe etwa 0,5 x 10¹⁰ Atome/cm² überschreitet.

9. Verfahren nach einem der Ansprüche 6 bis 8, bei dem die Spurenverunreinigung oder -verunreinigungen an Alkali-, Erdalkali- oder Übergangsmetall, auf die die abgezogene Probe analysiert wird, unter Natrium, Nickel, Eisen, Kupfer, Aluminium, Zink, Calcium, Titan, Kalium, Kobalt, Chrom, Molybdän und Mangan ausgewählt ist oder wird.

10. Verfahren nach einem der Ansprüche 6 bis 9, bei dem die Analyse der abgezogenen Probe automatisiert ist.

## Revendications

1. Procédé pour déterminer la concentration en une impureté ou en des impuretés métallique(s) dans une solution de nettoyage d'une plaquette de silicium, qui est maintenue en écoulement continu à travers un bain, le procédé comprenant:
l'introduction en continu à partir dudit écoulement d'une portion de la solution de bain de nettoyage dans et à travers une cellule échantillon;
le prélèvement d'un échantillon de la solution de nettoyage à partir de la cellule échantillon;
l'analyse de l'échantillon prélevé afin de déterminer la concentration en une impureté ou en des impuretés constituée(s) par des métaux alcalin, alcalino-terreux ou de transition à l'état de traces dans la solution de bain de nettoyage; et
la mise au rebut de la solution de nettoyage introduite dans la cellule échantillon.

2. Procédé selon la revendication 1, dans lequel l'impureté ou les impuretés constituée(s) par des métaux alcalin, alcalino-terreux ou de transition à l'état de traces, pour lesquelles l'échantillon prélevé est analysé, est ou sont choisie(s) parmi le sodium, le nickel, le fer, le cuivre, l'aluminium, le zinc, le calcium, le titane, le potassium, le cobalt, le chrome, le molybdène et le manganèse.

3. Procédé selon la revendication 1 ou la revendication 2, dans lequel l'analyse de l'échantillon prélevé est automatisée.

4. Procédé selon l'une quelconque des revendications 1 à 3, dans lequel la cellule échantillon est située à proximité de l'équipement de mesure analytique, de manière à ce que l'échantillon puisse être prélevé et analysé sans transporter la cellule échantillon vers l'équipement de mesure analytique.

5. Procédé selon l'une quelconque des revendications 1 à 4, dans lequel un échantillon est prélevé de la cellule échantillon et il est analysé à des intervalles compris entre environ 5 et environ 10 minutes.

6. Procédé pour déterminer si la concentration en une impureté ou en des impuretés métallique(s) à la surface d'une plaquette de silicium dépasse ou non une limite acceptable, le procédé comprenant:
l'immersion d'une plaquette de silicium dans une solution de nettoyage, qui est maintenue en écoulement continu à travers un bain;
l'introduction en continu à partir dudit écoulement d'une portion de la solution de bain de nettoyage dans et à travers une cellule échantillon;
le prélèvement d'un échantillon de la solution de nettoyage à partir de la cellule échantillon;
l'analyse de l'échantillon prélevé afin de déterminer la concentration en une impureté ou en des impuretés constituée(s) par des métaux alcalin, alcalino-terreux ou de transition à l'état de traces dans la solution de bain de nettoyage; et
la comparaison entre la concentration en une impureté ou en des impuretés dans la solution de nettoyage et une corrélation déterminée d'une manière empirique afin de déterminer si la concentration en une impureté ou en des impuretés à la surface de la plaquette de silicium dépasse environ 1 × 10¹⁰ atomes/cm².

7. Procédé selon la revendication 6, dans lequel la collecte et l'analyse de l'échantillon ont lieu alors que la plaquette est immergée dans la solution.

8. Procédé selon la revendication 6 ou la revendication 7, dans lequel on opère une comparaison entre l'impureté ou les impuretés dans la solution de nettoyage et la corrélation déterminée d'une manière empirique afin de déterminer si la concentration en l'impureté ou en les impuretés à la surface de la plaquette de silicium dépasse environ 0,5 × 10¹⁰ atomes/cm².

9. Procédé selon l'une quelconque des revendications 6 à 8, dans lequel l'impureté ou les impuretés constituée(s) par des métaux alcalin, alcalino-terreux ou de transition à l'état de traces, pour lesquelles l'échantillon prélevé est analysé, est ou sont choisie(s) parmi le sodium, le nickel, le fer, le cuivre, l'aluminium, le zinc, le calcium, le titane, le potassium, le cobalt, le chrome, le molybdène et le manganèse.

10. Procédé selon l'une quelconque des revendications 6 à 9, dans lequel l'analyse de l'échantillon prélevé est automatisée.
